# EUROPÄISCHE PATENTANMELDUNG

(11) **EP 4 659 665 A1**
(43) Veröffentlichungstag der Anmeldung: **10.12.2025**
(21) Anmeldenummer: 25176886.7
(22) Anmeldetag: 16.05.2025
(51) Int. Cl.: A61B 5/024, A61B 5/00

(54) **PATCH FÜR EINE ARMBANDUHR UND/ODER EIN ARMBAND, KIT, SYSTEM UND VERFAHREN**

(30) Priorität: 03.06.2024 DE 102024115393
(71) Anmelder: FORBENCAP GmbH, 87527 Sonthofen (DE)
(72) Erfinder: Götz, Prof. Dr. Stefan, 85659 Forstern (DE)
(74) Vertreter: advotec.

(57) **Zusammenfassung**

Patch (12) für eine Armbanduhr (10) und/oder ein Armband eines Benutzers, umfassend: Mittel zur insbesondere reversiblen Befestigung (26) an der Armbanduhr (10) und/oder dem Armband; mindestens einen Sensor (30) zur Erfassung mindestens einer Vitalfunktion des Benutzers; und vorzugsweise mindestens einen weiteren Sensor (32) zur Erfassung einer geografischen Position und/oder mindestens einer Beschleunigung des Benutzers.

## Beschreibung

Die Erfindung betrifft einen Patch für eine Armbanduhr und/oder ein Armband eines Benutzers. Die Erfindung betrifft ferner ein Kit umfassend einen Patch. Die Erfindung betrifft ferner ein System und ein Verfahren.

### Stand der Technik

Der Bereich Fitness und Körperbewusstsein gewinnt zunehmend an Bedeutung in unserer Gesellschaft. Was einst nur für den Hochleistungssportler von Interesse war, hat nun den Massenmarkt erreicht: die Nutzung von Smart Watches zur Überwachung von Vitalfunktionen.

Diese Smart Watches bieten eine Vielzahl von Funktionen, die weit über das bloße Anzeigen der Zeit hinausgehen, indem sie Herzfrequenz, Schlafmuster, Schritte und vieles mehr überwachen. Trotz ihrer Nützlichkeit erfüllen Smart Watches jedoch nicht immer die ästhetischen und modischen Anforderungen ihrer Nutzer.

Viele Menschen bevorzugen das Tragen klassischer mechanischer Uhren oder gar luxuri-öser antiker Zeitmesser, die einen besonderen Stil und Prestige verkörpern. Dies kann insbesondere auch bei prestigeträchtigen Sportarten wie Golf, Segeln oder Jagen gewünscht sein. Dieser Wunsch steht jedoch im Konflikt mit dem Interesse und ggf. auch einer gesundheitlichen Notwendigkeit, Vitalfunktionen insbesondere kontinuierlich überwachen zu können, da traditionelle Uhren diese Funktion nicht unterstützen. Somit muss sich der Nutzer zwischen der praktischen Funktionalität einer Smart Watch und der eleganten Ästhetik einer klassischen oder luxuriösen Uhr entscheiden.

Es ist eine Aufgabe der Erfindung, diese Problematik zu überwinden und einen alternativen Lösungsansatz anzugeben.

### Offenbarung der Erfindung

Die Aufgabe wird gelöst durch einen Patch gemäß den Merkmalen des Patentanspruchs 1. Die Aufgabe wird gelöst durch ein Kit gemäß den Merkmalen des Patentanspruchs 12. Die Aufgabe wird gelöst durch ein System gemäß den Merkmalen des Patentanspruchs 13. Die Aufgabe wird gelöst durch ein Verfahren gemäß den Merkmalen des Patentanspruchs 15.

Gemäß einem ersten Aspekt wird ein reversibel lösbar befestigbarer Patch für eine Armbanduhr und/oder ein Armband eines Benutzers vorgeschlagen. Der Patch umfasst Mittel zur insbesondere reversiblen Befestigung an der Armbanduhr und/oder dem Armband, mindestens einen Sensor zur Erfassung mindestens einer Vitalfunktion des Benutzers; und vorzugsweise mindestens einen weiteren Sensor zur Erfassung einer geografischen Position und/oder mindestens einer Beschleunigung des Benutzers.

Gemäß einem zweiten Aspekt wird ein Patch für eine Armbanduhr oder ein Schmuckarmband vorgeschlagen, wobei der Patch zumindest teilweise in einem reversibel lösbar befestigbaren Uhrenband der Armbanduhr oder einem reversibel lösbar befestigbaren Armband des Schmuckarmbandes integriert ist. Der Patch umfasst Mittel zur insbesondere reversiblen Befestigung an der Armbanduhr oder dem Schmuckarmband, mindestens einen Sensor zur Erfassung mindestens einer Vitalfunktion des Benutzers, und vorzugsweise mindestens einen weiteren Sensor zur Erfassung einer geografischen Position und/oder mindestens einer Beschleunigung des Benutzers.

Die für den Patch gemäß dem ersten Aspekt aufgeführten Merkmale gelten für den Patch gemäß dem zweiten Aspekt entsprechend. Der Patch gemäß dem zweiten Aspekt beschreibt eine alternative Ausführung zu dem Patch gemäß dem ersten Aspekt, da der Patch gemäß dem zweiten Aspekt nicht als zusätzliche Komponente an eine bestehende Uhr oder einem Schmuckarmband angebracht wird, sondern in einem Uhrenband oder einem Armband zumindest teilweise integriert wird. So kann ein Kunde einer Luxusuhr oder eines Schmuckarmbandes beispielsweise beim Hersteller ein Uhrenband oder ein Armband wählen, dass mindestens die vorliegend beschriebene Vitalfunktionsüberwachung bereitstellt. So kann der Kunde ein Luxusuhrenband oder ein Schmuckarmband wählen, dass äußerlich vom Stil nicht unterscheidbar zu einem "klassischen" Uhrenband ist, allerdings mindestens die Vitalfunktionsüberwachung bereitstellen kann.

Gemäß einem dritten Aspekt wird ein Kit vorgeschlagen, das mindestens einen solchen Patch und eine herkömmliche Armbanduhr, insbesondere und/oder ein Armband, an der/dem der Patch reversibel befestigbar ist, aufweist. Eine herkömmliche Armbanduhr ist vorliegend eine Uhr, die ein mechanisches Uhrwerk umfasst, vorzugsweise eine Luxusuhr. Die Armbanduhr und das Armband sind vorzugsweise nicht aus dem Smart-Vitality-Bereich, können also selbst keine Funktionen zur Überwachung eines Vitalparameters eines Benutzers bereitstellen, sondern müssen hierzu mit dem vorliegend vorgeschlagenen Patch ausgestattet werden. Es können auch zwei Patches an einer Armbanduhr oder einem Armband angebracht werden, um derart beispielsweise eine Mehrpunktauflage für das Messen von Vitalfunktionen, beispielsweise für die Pulsmessung, zu ermöglichen.

Gemäß einem vierten Aspekt wird ein System vorgeschlagen, das mindestens einen vorliegend beschriebenen Patch und ein externes Gerät aufweist, das dazu ausgebildet ist, die von dem Patch gesendeten Daten zu empfangen und/oder zu verarbeiten. Das externe Gerät kann beispielsweise ein Smartphone und/oder Tablet und/oder Laptop sein, das mit dem mindestens einen Patch, insbesondere kommunikativ, koppelbar sein kann.

Gemäß einem fünften Aspekt wird ein Verfahren zur Überwachung von mindestens einer Vitalfunktion und/oder einem Standort eines Benutzers vorgeschlagen. Das Verfahren umfassend die Schritte:
a) Bereitstellen mindestens eines vorliegend vorgeschlagenen Patches an einer Armbanduhr und/oder einem Armband des Benutzers;
b) Erfassen der mindestens einen Vitalfunktion und/oder des Standortes des Benutzers durch den mindestens einen Patch; und
c) Senden der erfassten Daten an ein externes Gerät.

Das Bereitstellen des mindestens einen Patches kann ein Befestigen oder Anbringen des mindestens einen Patches aufweisen. Das Befestigen ist vorzugsweise reversibel lösbar möglich. Das Erfassen der mindestens einen Vitalfunktion wird vorzugsweise durch mindestens einen in dem Patch umfassten Sensor bereitgestellt. Das Senden der Daten des Patches kann über eine optische und/oder eine Funkschnittstelle, beispielsweise eine Bluetooth-Schnittstelle, eine WLAN-Schnittstelle, eine Mobilfunk-Schnittstelle und/oder eine NRF-Schnittstelle erfolgen. Der Patch kann auch zum Empfangen von Daten von dem externen Gerät eingerichtet sein. Diese Daten können beispielsweise Konfigurationsdaten und/oder Software-Update-Daten aufweisen.

Das Merkmal "reversibel lösbar befestigbarer Patch" beschreibt vorzugsweise, dass der Patch so konzipiert ist, dass er durch einen Benutzer auf einfache und intuitive Weise an einer Armbanduhr oder einem Armband befestigt und auch wieder entfernt werden kann. Dies ermöglicht eine flexible Nutzung, je nach Bedarf des Benutzers, ohne dauerhafte Veränderungen an der Armbanduhr oder dem Armband vornehmen zu müssen.

Alternativ kann der Patch auch in dem Uhrenband oder einem Armband eines Schmuckarmbandes zumindest teilweise integriert sein, um derart ein äußeres Erscheinungsbild der Uhr oder der Schmuckarmbandes nicht zu verändern. Der Kunde bzw. Benutzer kann den so ausgestalten Patch dann beispielsweise als Zusatzfeature bei der Auswahl und/oder Konfiguration der Uhr oder des Schmuck-Armbandes dazuwählen.

Das Merkmal "Mittel zur insbesondere reversiblen Befestigung" bezieht sich auf eine mechanische und/oder magnetische Vorrichtung oder auf ein sonstiges Befestigungsmittel, beispielsweise ein Haftstoff oder eine Anhaftschicht, die es ermöglichen, den Patch an der Armbanduhr oder dem Armband zu befestigen und ihn bei Bedarf wieder zu entfernen. Diese Mittel können eine Art von Klettverschluss, Clips und/oder Magnete umfassen. Auch Klebeschichten, beispielsweise auf Silikonbasis, sind vorstellbar. Die Mittel zum Befestigen können an dem Patch befestigt, auf diesem aufgebracht oder in diesem integriert sein.

Wenn der Patch in dem Uhrenband oder dem Armband zumindest teilweise integriert ist, können die Mittel zum Befestigen auch mindestens einen Pin oder eine Verschraubung oder einen Magnetverschluss oder sonstige "klassische" Mittel zum Befestigen eines Uhrenbandes oder eines Armbandes eines Schmuckarmbandes aufweisen.

Das Merkmal "mindestens einen Sensor zur Erfassung mindestens einer Vitalfunktion des Benutzers" beschreibt, dass der Patch mindestens einen Sensor aufweist, der in der Lage ist, mindestens eine Vitalfunktion wie eine Herzfrequenz, einen Blutdruck, eine Sauerstoffsättigung und/oder eine Hauttemperatur des Benutzers zu überwachen. Dieser mindestens eine Sensor lieft vorzugsweise Gesundheitsdaten, die kontinuierlich oder bei Bedarf erfasst werden können.

Das Merkmal "mindestens einen weiteren Sensor zur Erfassung einer geografischen Position und/oder mindestens einer Beschleunigung des Benutzers" beschreibt, dass der Patch zusätzlich zu dem mindestens einen Vitalfunktionssensor auch mindestens einen weiteren Sensor aufweisen kann, der geografische Daten (wie GPS und/oder andere GNSS) und/oder Beschleunigungsdaten (linear und/oder rotatorisch) erfassen kann. Dieser Sensor kann dabei helfen, die Bewegungen des Benutzers zu verfolgen und seine Position zu bestimmen, was besonders nützlich für Fitness-Tracking und Navigationszwecke ist.

Der Patch bietet eine flexible und nicht-invasive Möglichkeit, traditionelle und luxuriöse Armbanduhren um moderne Vital- und Tracking-Funktionen zu erweitern, ohne auf die ästhetischen Vorteile klassischer Uhren verzichten zu müssen. Er ermöglicht es dem Benutzer, sowohl seine Vitalität bzw. Gesundheit im Blick zu behalten als auch seinen persönlichen Stil zu bewahren.

Die für den Patch gemachten Ausführungen gelten für das Kit, das System und das Verfahren entsprechend. Dabei versteht es sich, dass sprachliche Abwandlungen von Merkmalen nach sprachüblicher Praxis beispielsweise für das Verfahren umformulierbar sind, ohne dass derartige Formulierungen explizit hier aufgeführt werden müssen.

In einem weiteren Aspekt wird vorgeschlagen, dass das Mittel zur reversiblen Befestigung durch einen Klettverschluss und/oder magnetisch und/oder mechanisch und/oder adhäsiv und/oder kohäsiv an der Armbanduhr und/oder dem Armband befestigbar ist.

Der Patch kann mit einem Klettverschluss versehen sein, der es ermöglicht, ihn einfach und sicher an der Armbanduhr oder dem Armband zu befestigen und bei Bedarf wieder zu entfernen. Klettverschlüsse bieten eine starke Haftung und sind gleichzeitig leicht zu handhaben. Alternativ oder zusätzlich kann der Patch magnetische Elemente enthalten, die ihn sicher an der Uhr oder dem Armband halten. Magnetische Befestigungen sind besonders praktisch, da sie eine schnelle und unkomplizierte Anbringung und Entfernung ermöglichen, ohne mechanische Verschleißteile. Eine weitere Möglichkeit besteht darin, den Patch mit einer adhäsiven Oberfläche auszustatten. Dies könnte eine klebende Substanz oder spezielle Klebefolien umfassen, die eine starke, aber dennoch rückstandsfreie Haftung gewährleisten. Diese Methode ermöglicht eine sichere Befestigung auf verschiedenen Materialien und Oberflächen. Schließlich kann der Patch auch kohäsive Materialien verwenden, die ohne zusätzliche Klebstoffe durch Molekular- oder Oberflächenkräfte aneinander haften. Diese Methode bietet eine wiederverwendbare und zuverlässige Befestigung, die leicht zu lösen und wieder zu befestigen ist. Diese verschiedenen Befestigungsmethoden gewährleisten, dass der Patch flexibel und sicher an verschiedenen Arten von Armbanduhren und Armbändern angebracht werden kann, ohne deren Aussehen oder Funktion zu beeinträchtigen. Der Benutzer kann somit von den Vorteilen der Vitalfunktionenüberwachung profitieren, während er seine bevorzugte Uhr oder sein bevorzugtes Armband trägt. Der Patch kann auch durch mechanische Mittel, beispielsweise eine Schraube und/oder einen Bolzen und/oder einen Stift usw. befestigbar sein, insbesondere wenn er zumindest teilweise in dem Uhrenband und/oder dem Armband integriert ist.

In einem weiteren Aspekt wird vorgeschlagen, dass der Patch ein System on a Chip (SoC) oder einen Mono- oder Mehrkern-Prozessor umfasst.

Ein SoC ist ein hochintegriertes elektronisches Bauteil, das mehrere Funktionen auf einem einzigen Chip vereint und somit die Effizienz und Leistungsfähigkeit des Patches erheblich steigert. Durch die Integration eines SoC können sämtliche elektronischen Komponenten, die zur Überwachung von Vitalfunktionen und zur Positionsbestimmung notwendig sind, auf minimalem Raum untergebracht werden. Dies ermöglicht ein schlankes und leichtes Design des Patches. Das SoC umfasst vorzugsweise einen leistungsfähigen Mikrocontroller, der in der Lage ist, Daten von verschiedenen Sensoren zu verarbeiten und zu analysieren. Dadurch kann der Patch präzise und zuverlässige Informationen über den Gesundheitszustand und die Bewegungen des Benutzers liefern. Das SoC ermöglicht die Integration mehrerer Sensoren auf einem einzigen Chip. Dies umfasst Sensoren zur Erfassung von Vitalfunktionen wie Herzfrequenz, Blutdruck und Hauttemperatur sowie Sensoren zur Positionsbestimmung (GPS) und zur Erfassung von Beschleunigung. Diese umfassende Sensorik sorgt dafür, dass der Patch eine breite Palette an Daten erfassen und auswerten kann. Dank des SoC ist der Patch in der Lage, sehr energieeffizient zu arbeiten. Das SoC umfasst Energiemanagement-Funktionen, die den Energieverbrauch minimieren und somit die Batterielaufzeit des Patches verlängern. Dies ist besonders wichtig für die kontinuierliche Überwachung von Vitalfunktionen über längere Zeiträume. Das SoC ermöglicht auch die drahtlose Kommunikation mit anderen Geräten, wie Smartphones oder Computern. Dadurch können die gesammelten Daten einfach und schnell übertragen und analysiert werden, ohne dass eine physische Verbindung erforderlich ist. Durch die Integration eines Systems on a Chip wird der Patch zu einem leistungsstarken, vielseitigen und dennoch kompakten Gerät, das eine nahtlose Überwachung der Vitalfunktionen und der Position des Benutzers ermöglicht. Gleichzeitig bleibt er diskret und beeinträchtigt nicht den Tragekomfort oder die Ästhetik von Armbanduhren und Armbändern.

Ein Monokern-Prozessor, auch Einkern-Prozessor genannt, ist vorzugsweise ein Prozessor, der nur einen einzelnen Verarbeitungskern enthält. Dieser Kern ist für die Ausführung von Befehlen und die Verarbeitung von Daten verantwortlich. In einem Monokern-Prozessor erfolgt die gesamte Verarbeitung vorzugsweise sequenziell, was bedeutet, dass er immer nur eine Aufgabe zur selben Zeit bearbeiten kann. Da beispielsweise nur ein Kern vorhanden ist, sind die Möglichkeiten zur parallelen Verarbeitung begrenzt, was die Leistung bei gleichzeitiger Ausführung mehrerer Aufgaben einschränkt. Die Architektur von Monokern-Prozessoren ist im Vergleich zu Mehrkern-Prozessoren vorzugsweise einfacher, was sie kostengünstiger und weniger komplex in der Herstellung macht.

Mehrkern-Prozessoren, auch Multikern-Prozessoren genannt, sind vorzugsweise Prozessoren, die zwei oder mehr Verarbeitungskerne auf einem einzigen Chip integrieren. Jeder dieser Kerne kann vorzugsweise unabhängig voneinander Aufgaben ausführen, was zu einer erheblichen Steigerung der Gesamtleistung und Effizienz des Prozessors führt. Im Gegensatz zu Monokern-Prozessoren, die nur einen Kern haben und daher nur eine Aufgabe zur gleichen Zeit bearbeiten können, ermöglichen Mehrkern-Prozessoren vorzugsweise die parallele Verarbeitung mehrerer Aufgaben. Mehrere Kerne ermöglichen die gleichzeitige Ausführung mehrerer Aufgaben, was die Multitasking-Fähigkeiten eines Systems verbessert. Durch die parallele Verarbeitung können Anwendungen, die für Multithreading optimiert sind, die Leistung der Kerne voll ausschöpfen und somit schneller und effizienter arbeiten. Mehrkern-Prozessoren können bei gleicher oder sogar geringerer Leistungsaufnahme mehr Rechenleistung bieten als ein einzelner schnellerer Kern.

Der Patch kann besonders bevorzugt als ein- oder mehrschichtiges Printed-Circuit-Board (PCB) oder auch als ein- oder mehrschichtiges Flexible Printed Circuit Board (FPCB) ausgebildet sein. Der als SoC und/oder PCB und/oder FPCB ausgebildete Patch kann vorzugsweise in einem insbesondere hautneutralen bzw. hautfreundlichen Kunststoff oder Silikon zumindest teilweise eingebettet sein.

In einem weiteren Aspekt wird vorgeschlagen, dass das SoC eine Verarbeitungseinheit, eine Speichereinheit und/oder eine Kommunikationseinheit und/oder eine Energieversorgungseinheit aufweist.

Die Verarbeitungseinheit ist vorzugsweise das "Herzstück" des SoC. Sie umfasst vorzugsweise einen Mikrocontroller oder Mikroprozessor, der die Daten von den verschiedenen Sensoren verarbeitet und analysiert. Diese Verarbeitungseinheit sorgt dafür, dass der Patch präzise und zuverlässige Informationen über die Vitalfunktionen und Bewegungen des Benutzers liefert. Die Speichereinheit innerhalb des SoC dient vorzugsweise zur (Zwischen-) Speicherung der erfassten Daten. Sie kann sowohl flüchtigen (RAM) als auch nichtflüchtigen Speicher (Flash-Speicher) umfassen, um sowohl temporäre als auch dauerhafte Daten zu sichern. Dies ermöglicht es dem Patch, eine Historie der Vitaldaten zu führen und diese bei Bedarf abzurufen. Die durch den Patch erfassten Daten können auch an ein Endgerät übertragen werden, um so den Speicherbedarf auf dem Patch gering zu halten. Die Kommunikationseinheit ermöglicht die drahtlose Übertragung der gesammelten Daten an andere Geräte, wie Smartphones oder Computer. Diese Einheit kann verschiedene Kommunikationsprotokolle unterstützen, einschließlich Bluetooth, WLAN oder NFC, um eine nahtlose und flexible Datenübertragung zu gewährleisten. Die Energieversorgungseinheit ist dafür verantwortlich, den Patch mit der notwendigen Energie zu versorgen. Sie umfasst sowohl die Batteriemanagementsysteme als auch mögliche Energieerzeugungs- oder
-speicherlösungen. Diese Einheit sorgt dafür, dass das SoC energieeffizient arbeitet und die Batterielaufzeit maximiert wird, was besonders wichtig für die kontinuierliche Überwachung der Vitalfunktionen ist. Durch die Integration dieser verschiedenen Einheiten in das SoC wird der Patch zu einem leistungsstarken und vielseitigen Gerät. Es ermöglicht eine präzise und zuverlässige Überwachung der Vitalfunktionen des Benutzers, eine effiziente Datenverarbeitung und -speicherung sowie eine flexible und drahtlose Datenübertragung, während gleichzeitig eine energieeffiziente Betriebsweise sichergestellt wird. Der Patch bleibt dabei kompakt und beeinträchtigt nicht den Tragekomfort oder die Ästhetik von Armbanduhren und Armbändern.

In einem weiteren Aspekt wird vorgeschlagen, dass der Patch autonom und/oder drahtlos energetisch versorgbar ist. In einer besonders bevorzugten Ausführungsform umfasst der Patch für eine drahtlose Energieversorgung eine elektrisch leitfähige Spule, die ferner eine elektronische Gleichrichtereinheit mit Dioden und/oder Transistoren umfassen kann. Die Induktivität der Spule kann vor dem elektronischen Gleichrichter teilweise oder vollständig durch einen Serienkondensator kompensiert sein.

Der Patch ist vorzugsweise so konzipiert, dass er eigenständig, also autonom, betrieben werden kann. Dies bedeutet, dass er eine eigene Energiequelle, wie eine integrierte Batterie, besitzt, die den Betrieb der verschiedenen Sensoren und des Systems on a Chip (SoC) ermöglicht. Diese Autonomie stellt sicher, dass der Patch unabhängig von externen Energiequellen funktioniert und somit jederzeit und überall einsatzbereit ist. Alternativ oder ergänzend kann der Patch auch drahtlos energetisch versorgt werden. Diese drahtlose Energieversorgung kann durch Technologien wie Induktionsladen oder Solarenergie erfolgen. Bei der Induktionsladung wird der Patch auf eine spezielle Ladefläche gelegt, die die Energie drahtlos überträgt und die Batterie des Patches auflädt. Solarzellen könnten ebenfalls in den Patch integriert werden, um die Energie aus der Umgebung zu nutzen und somit eine nachhaltige und kontinuierliche Energieversorgung zu gewährleisten. Durch diese autonomen und drahtlosen Energieversorgungsmöglichkeiten wird der Patch äußerst flexibel und praktisch im täglichen Gebrauch. Der Benutzer muss sich keine Sorgen über häufiges Aufladen oder den Austausch von Batterien machen, da der Patch durch seine eigenständige und drahtlose Energieversorgung stets betriebsbereit ist. Diese Merkmale tragen erheblich zur Langlebigkeit und Zuverlässigkeit des Patches bei, was ihn zu einer idealen Lösung für die kontinuierliche Überwachung von Vitalfunktionen und Bewegungen macht.

In einem weiteren Aspekt wird vorgeschlagen, dass der Patch Mittel zum Aufladen einer internen Energieversorgungseinheit aufweist.

Der Patch ist vorzugsweise mit einer internen Energieversorgungseinheit ausgestattet, vorzugsweise einer wiederaufladbaren Batterie. Um diese Batterie effizient aufladen zu können, enthält der Patch spezielle Mittel zum Aufladen. Diese Mittel können verschiedene Technologien umfassen. Eine Möglichkeit ist die Integration von Induktionsladetechnologie, die es ermöglicht, den Patch drahtlos aufzuladen. Hierbei wird der Patch einfach auf eine dafür vorgesehene Ladefläche gelegt, die Energie über Induktion überträgt und die interne Batterie des Patches auflädt. Diese Technologie bietet eine bequeme und benutzerfreundliche Möglichkeit, den Patch aufzuladen, ohne dass Kabel oder Steckverbindungen erforderlich sind. Zusätzlich oder alternativ könnte der Patch auch über einen herkömmlichen Ladeanschluss verfügen, der das Aufladen der internen Batterie mittels eines Kabels ermöglicht. Diese Methode bietet eine zuverlässige und schnelle Auflademöglichkeit, insbesondere wenn drahtlose Ladegeräte nicht verfügbar sind. Durch die Bereitstellung von Mitteln zum Aufladen der internen Energieversorgungseinheit stellt der Patch sicher, dass seine Sensoren und das System on a Chip (SoC) kontinuierlich mit Energie versorgt werden. Dies gewährleistet eine ununterbrochene Funktionalität, sodass der Benutzer jederzeit und überall auf die wichtigen Gesundheits- und Bewegungsdaten zugreifen kann.

In einem weiteren Aspekt wird vorgeschlagen, dass der Patch mehrere Sensoren zur Erfassung unterschiedlicher Vitalfunktionen umfasst.

Der Patch kann einen Herzfrequenzsensor aufweisen. Der Herzfrequenzsensor misst vorzugsweise kontinuierlich die Herzfrequenz des Benutzers und liefert Informationen über die kardiovaskuläre Gesundheit. Er kann Unregelmäßigkeiten erkennen und Trends über die Zeit verfolgen. Der Patch kann einen Blutdrucksensor aufweisen. Dies ist besonders nützlich für Personen mit Bluthochdruck oder anderen kardiovaskulären Erkrankungen, da es ihnen ermöglicht, ihren Blutdruck regelmäßig zu überwachen und potenziell gefährliche Veränderungen frühzeitig zu erkennen. Der Patch kann einen Temperatursensor aufweisen. Dieser Sensor misst die Körpertemperatur des Benutzers. Eine ansteigende Körpertemperatur kann auf eine Infektion oder Entzündung hinweisen, während eine zu niedrige Temperatur auf andere gesundheitliche Probleme hindeuten kann. Der Patch kann ein Pulsoximeter aufweisen. Ein Pulsoximeter misst den Sauerstoffgehalt im Blut. Dies ist besonders wichtig für Personen mit Atemwegserkrankungen wie Asthma oder COPD, da es ihnen hilft, ihre Sauerstoffsättigung zu überwachen. Der Patch kann ein Elektrokardiogramm (EKG)-Sensor aufweisen. Ein EKG-Sensor kann detaillierte Informationen über die Herzaktivität des Benutzers liefern. Er kann dabei helfen, Herzprobleme wie Arrhythmien oder Herzinfarkte frühzeitig zu erkennen. Vorzugsweise umfasst ein EKG-Sensor eine Messung der elektrischen Erregung des Herzens mit geeignetem elektronischem Messverstärker. Ferner kann ein EKG-Sensor einen Beschleunigugnssensor vorzugsweise in der Nähe einer Arterie, beispielsweise der arteriae radialis oder ulnaris, umfassen. Diese verschiedenen Sensoren sind vorzugsweise in den Patch integriert und arbeiten vorzugsweise zusammen, um eine umfassende Überwachung der Vitalfunktionen des Benutzers zu ermöglichen. Die gesammelten Daten können analysiert und bei Bedarf mit Gesundheitsdiensten und/oder Anwendungen auf mobilen Geräten synchronisiert werden, um eine kontinuierliche und präzise Gesundheitsüberwachung zu gewährleisten. Durch die Integration mehrerer Sensoren zur Erfassung unterschiedlicher Vitalfunktionen bietet der Patch eine umfassende Lösung für die Gesundheitsüberwachung. Er ermöglicht es dem Benutzer, seine Gesundheit ganzheitlich im Blick zu behalten und bei Bedarf schnell auf Veränderungen zu reagieren.

In einem weiteren Aspekt wird vorgeschlagen, dass mindestens einer der Sensoren einen Herzfrequenzsensor und/oder einen Blutsauerstoffsättigungssensor (bspw. optische Pulsoximetrie) und/oder einen Beschleunigungssensor und/oder mindestens eine Elektrode und einen Verstärker und/oder mindestens eine Photodiode und/oder einen Haut-/Gewebeimpedanzsensor aufweist, und/oder wobei der mindestens eine weitere Sensor einen Standortsensor, insbesondere ein GPS-Modul, und/oder einen Beschleunigungssensor aufweist.

Herzsignale lassen sich vorzugsweise elektrisch über Elektroden und Verstärker oder per Beschleunigungssensoren messen. Alternativ lassen sich Herzsignale auch optisch über die Pulsation eines Rücksignals in der Photodiode und vorzugsweise so auch als Nebenprodukt in der Pulsoximetrie messen. Ein Beschleunigungssensor erfasst die Bewegungen und Beschleunigungen des Benutzers. Dies ist nützlich für die Überwachung von Aktivitäten, zur Erkennung von Stürzen und zur Analyse von Bewegungsmustern. Eine Elektrode und ein Verstärker sind vorzugsweise Teil eines Elektrokardiogramm (EKG)-Sensors, der detaillierte Informationen über die Herzaktivität des Benutzers liefert. Der Verstärker verstärkt die elektrischen Signale des Herzens, die von der Elektrode erfasst werden, um eine genaue Analyse zu ermöglichen. Eine Photodiode wird vorzugsweise in Kombination mit einem Lichtemitter in optischen Sensoren verwendet, um die Herzfrequenz und die Blutsauerstoffsättigung zu messen. Sie detektiert das vom Gewebe reflektierte Licht und ermöglicht so die Berechnung dieser Vitalparameter. Eine Haut-/Gewebeimpedanzsensor misst vorzugsweise die elektrische Impedanz der Haut oder des Gewebes. Dies kann für die Bestimmung des Hydrationsstatus, der Körperzusammensetzung und anderer physiologischer Parameter nützlich sein. Ein GPS-Modul ermöglicht die präzise Bestimmung der geografischen Position des Benutzers. Dies ist besonders nützlich für Outdoor-Aktivitäten, Navigation und das Tracking von Lauf- oder Fahrradrouten.

In einem weiteren Aspekt wird vorgeschlagen, dass der Patch innenseitig an der Armbanduhr und/oder dem Armband, insbesondere in Richtung einer Handgelenkinnenseite oder einer Handgelenkaußenseite befestigbar ist.

Der Patch ist vorzugsweise so konzipiert, dass er auf der Innenseite der Armbanduhr oder des Armbands angebracht werden kann, insbesondere in Richtung der Handgelenkinnenseite oder der Handgelenkaußenseite. Wenn der Patch an der Innenseite des Handgelenks befestigt wird, liegt er direkt auf der Haut des Benutzers. Dies ermöglicht eine präzise Erfassung der Vitalfunktionen wie Herzfrequenz und Blutsauerstoffsättigung, da die Sensoren in direktem Kontakt mit der Haut stehen. Die Handgelenkinnenseite bietet zudem eine stabile Position, die Bewegungsartefakte minimiert und so die Genauigkeit der Messungen erhöht. Alternativ oder ergänzend kann der Patch auch an der Außenseite des Handgelenks befestigt werden. Dies kann aus ästhetischen Gründen oder zur besseren Integration in das Design der Armbanduhr bevorzugt werden. Auch auf der Außenseite kann der Patch zuverlässig arbeiten, besonders wenn es um die Erfassung von Bewegungsdaten durch Beschleunigungssensoren oder die Bestimmung der geografischen Position mittels GPS geht. Die innenseitige Befestigung des Patches stellt sicher, dass die Sensoren optimal positioniert sind, um genaue und zuverlässige Daten zu erfassen, während sie gleichzeitig den Tragekomfort des Benutzers nicht beeinträchtigen. Diese flexible Platzierungsmöglichkeit ermöglicht es, den Patch diskret und effizient in den täglichen Gebrauch zu integrieren, ohne das ästhetische Erscheinungsbild der Armbanduhr oder des Armbands zu beeinträchtigen.

In einem weiteren Aspekt wird vorgeschlagen, dass der Patch dazu eingerichtet ist, über einen Energietransfer von der Armbanduhr und/oder dem Armband betreibbar zu sein, oder wobei der Patch dazu eingerichtet ist, seine Energie durch Energyharvesting von der Armbanduhr und/oder dem Armband zu beziehen.

Der Patch kann seine Energie vorzugsweise direkt von der Armbanduhr oder dem Armband beziehen. Dies könnte durch eine direkte elektrische Verbindung oder durch drahtlose Technologien wie Induktionsladung geschehen. Diese Methode stellt sicher, dass der Patch kontinuierlich mit Energie versorgt wird, solange die Armbanduhr oder das Armband selbst über eine Energiequelle verfügt. Diese nahtlose Integration ermöglicht eine zuverlässige und unterbrechungsfreie Funktion des Patches. Alternativ oder zusätzlich kann der Patch so konzipiert sein, dass er seine Energie durch Energy-Harvesting von der Armbanduhr und/oder dem Armband bezieht. Dies bedeutet, dass der Patch Energie aus seiner Umgebung sammelt und nutzt, um sich selbst zu betreiben. Mögliche Energy-Harvesting-Methoden umfassen die Nutzung von kinetischer Energie, die durch die Bewegungen des Benutzers erzeugt wird, oder die Umwandlung von Umgebungslicht in elektrische Energie durch kleine Solarzellen. Diese Technologien ermöglichen es dem Patch, unabhängig von externen Energiequellen zu arbeiten, was seine Einsatzmöglichkeiten und Flexibilität erheblich erweitert. Eine Möglichkeit zum Energy-Harvesting kann ein Seebeckgenerator zur Energieentnahme aus Wärmestrom zwischen Arm und Armbanduhr sein, da eine hohe Wärmekapazität und Entwärmungsfläche zur Umgebung bereitgestellt wird. Eine weitere Möglichkeit zum Energy-Harvesting kann eine rotierend gelagerte Masse mit hoher Unwucht zusammen mit rotatorischem Synchron- oder Asynchrongenerator sein. Eine weitere Möglichkeit kann eine linear bewegliche Masse mit einem Lineargenerator sein.

In einem weiteren Aspekt wird vorgeschlagen, dass der Patch ferner eine drahtlose Kommunikationseinheit umfasst, um Daten an ein externes Gerät zu senden und/oder von dem externen Gerät zu empfangen, wobei das externe Gerät ein Mobiltelephon und/oder eine Cloud und/oder ein Server und/oder eine mobil Rechen- und/oder Steuereinrichtung umfassen kann.

Der Patch ist vorzugsweise mit einer drahtlosen Kommunikationseinheit ausgestattet, die verschiedene drahtlose Kommunikationsstandards unterstützen kann, wie beispielsweise Bluetooth, WLAN oder andere geeignete Technologien. Diese Einheit ermöglicht es dem Patch, die erfassten Daten in Echtzeit oder periodisch an ein externes Gerät zu übertragen. Der Patch kann Daten an ein Smartphone senden, das dann als zentrale Einheit zur Anzeige und Analyse der gesammelten Vital- und Bewegungsdaten dient. Über eine entsprechende App können Benutzer ihre Gesundheitsinformationen überwachen, analysieren und gegebenenfalls mit Gesundheitsdienstleistern teilen. Der Patch kann Daten direkt an eine Cloud-Plattform senden. Dies ermöglicht die Speicherung und Analyse der Daten auf entfernten Servern, wodurch Benutzer von überall aus auf ihre Gesundheitsinformationen zugreifen können. Dies ist besonders nützlich für langfristige Gesundheitsüberwachung und die Nutzung fortschrittlicher Analysen und Algorithmen. Alternativ oder ergänzend können die Daten an einen spezifischen Server gesendet werden, der für die Speicherung und Verarbeitung der Gesundheitsdaten vorgesehen ist. Dies kann für institutionelle Anwendungen, wie in Krankenhäusern oder Forschungszentren, von Vorteil sein. Der Patch kann auch Daten an mobile Rechen- und Steuereinrichtungen senden, die speziell für die Verarbeitung und Analyse von Gesundheitsdaten entwickelt ist. Diese Geräte können fortschrittliche Algorithmen nutzen, um Echtzeit-Feedback und Gesundheitswarnungen zu geben. Die drahtlose Kommunikationseinheit des Patches stellt sicher, dass die erfassten Daten effizient und sicher übertragen werden können, ohne dass physische Verbindungen erforderlich sind. Dies erhöht die Benutzerfreundlichkeit und Flexibilität des Patches erheblich, da er nahtlos mit verschiedenen externen Geräten interagieren kann, um eine umfassende Gesundheitsüberwachung zu gewährleisten. Durch die Integration dieser drahtlosen Kommunikationseinheit bietet der Patch eine Lösung zur Überwachung der Vitalfunktionen und Bewegungen des Benutzers. Er ermöglicht eine einfache und effektive Datenübertragung, die eine tiefgehende Analyse und kontinuierliche Gesundheitsüberwachung unterstützt, und verbessert somit die Gesamtfunktionalität und den Nutzen des Patches erheblich.

In einem weiteren Aspekt wird vorgeschlagen, dass das externe Endgerät (des Systems) ferner eine Benutzeroberfläche umfasst, die dem Benutzer ermöglicht, die Funktionalität des Patches zu steuern oder die erfassten Daten anzuzeigen.

Das externe Endgerät, das mit dem Patch kommuniziert, kann ein Smartphone, ein Tablet, ein Computer oder ein anderes geeignetes Gerät sein. Über die Benutzeroberfläche des externen Endgeräts kann der Benutzer die verschiedenen Funktionen des Patches steuern. Dies umfasst die Aktivierung oder Deaktivierung bestimmter Sensoren, das Starten oder Stoppen von Messungen sowie die Anpassung von Einstellungen wie Messintervallen und Alarmgrenzen. Diese Steuerung erfolgt typischerweise über eine intuitive App oder Software, die auf dem externen Endgerät installiert ist. Die Benutzeroberfläche des externen Endgeräts zeigt die vom Patch gesammelten Vital- und Bewegungsdaten an. Dies kann in Form von Echtzeit-Daten, historischen Trends oder zusammengefassten Berichten geschehen. Die Daten werden in leicht verständlichen Formaten wie Grafiken, Diagrammen oder numerischen Werten dargestellt, sodass der Benutzer einen klaren Überblick über seine Gesundheitsparameter erhält. Die Benutzeroberfläche kann auch interaktives Feedback bieten, wie Benachrichtigungen oder Warnungen, wenn bestimmte Gesundheitsparameter außerhalb des normalen Bereichs liegen. Der Benutzer kann sofort auf diese Benachrichtigungen reagieren und notwendige Maßnahmen ergreifen. Die Benutzeroberfläche ist so gestaltet, dass sie benutzerfreundlich und leicht navigierbar ist. Sie ermöglicht es dem Benutzer, die Anzeige und Steuerung der Daten nach seinen persönlichen Vorlieben anzupassen. Dies kann die Auswahl bestimmter Datenpunkte, die Art der Darstellung und die Häufigkeit der Aktualisierungen umfassen. Durch die Integration einer Benutzeroberfläche auf dem externen Endgerät wird die Interaktion mit dem Patch erheblich verbessert. Der Benutzer erhält eine zentrale Plattform zur Verwaltung und Analyse der Gesundheitsdaten sowie zur Steuerung der Patch-Funktionen. Dies erleichtert die Überwachung und Verwaltung der persönlichen Gesundheitsdaten und trägt dazu bei, dass der Benutzer fundierte Entscheidungen über seine Gesundheit treffen kann.

In einem weiteren Aspekt wird vorgeschlagen, dass der Patch ein thermisch reaktives Material aufweist, das in Abhängigkeit von der Hauttemperatur des Benutzers seine Form und/oder Elastizität verändert, um die Haftung an der Armbanduhr oder dem Armband zu optimieren. Dadurch kann sich der Patch automatisch an unterschiedliche Umgebungsbedingungen und Hauttemperaturen anpassen und somit eine verbesserte und komfortable Anbindung sicherstellen. Die thermisch induzierte Anpassung ermöglicht eine stabilere und hautnähere Lage, ohne dass zusätzliche mechanische Mittel erforderlich sind.

In einem weiteren Aspekt wird vorgeschlagen, dass die Sensoren des Patches, insbesondere der Sensor zur Erfassung von Vitalfunktionen und der Sensor zur Erfassung einer Position und/oder Beschleunigung, in mindestens zwei elektrisch voneinander getrennten Sensoreinheiten segmentiert sind, die in unterschiedlichen Ebenen eines mehrschichtigen FPCB angeordnet sind. Diese geschichtete oder segmentierte Anordnung bewirkt eine funktionale Trennung der Signalwege und verbessert die Signalqualität durch Reduktion potenzieller Störeinflüsse zwischen den Messsystemen.

In einem weiteren Aspekt wird vorgeschlagen, dass der Patch ein kapazitives oder optisches System zur automatischen Erkennung des Tragens auf der Haut aufweist und in einem inaktiven Energiesparmodus verbleibt, solange keine Hautdetektion erfolgt. Diese Trageerkennung erlaubt eine gezielte Aktivierung der Sensorik nur bei tatsächlichem Hautkontakt, wodurch der Energieverbrauch reduziert und die Lebensdauer der Energiequelle verlängert werden kann.

In einem weiteren Aspekt wird vorgeschlagen, dass das Flexible Printed Circuit Board (FPCB) des Patches eine Biegeflexibilität von höchstens 0,5 N·mm und eine Rückstellkraft von mindestens 70 % innerhalb von fünf Sekunden nach einer Verformung aufweist. Durch dieses definierte Verhältnis von Flexibilität und Rückstellkraft wird ein hoher Tragekomfort bei gleichzeitiger Formstabilität gewährleistet, was insbesondere bei wiederholtem Anlegen des Patches von Vorteil ist.

In einem weiteren Aspekt wird vorgeschlagen, dass die Datenverbindung zwischen dem Patch und einem externen Gerät durch ein mehrstufiges Authentifizierungsverfahren abgesichert ist, das mindestens eine lokale biometrische Verifikation und eine Cloud-basierte Identitätsprüfung umfasst. Durch diese Mehrpunkt-Authentifizierung wird ein besonders hoher Datenschutzstandard erreicht, der den Einsatz des Patches auch im medizinischen Umfeld oder bei besonders sensiblen personenbezogenen Daten ermöglicht.

In einem weiteren Aspekt wird vorgeschlagen, dass der Patch in einem transparenten oder transluzenten Silikonmaterial eingebettet ist, das eine optische Schnittstelle zur Datenübertragung mittels Lichtsignalen, beispielsweise im Infrarotbereich, ermöglicht. Diese transparente Einbettung erlaubt eine drahtlose optische Kommunikation über Lichtsignale, beispielsweise mit Hilfe von integrierten Photodioden, ohne dass eine elektromagnetische Verbindung erforderlich ist.

In einem weiteren Aspekt wird vorgeschlagen, dass der Patch eine integrierte Abschirmstruktur zur Reduktion elektromagnetischer Störsignale aufweist. Die Abschirmung kann beispielsweise durch eine leitfähige Außenschicht oder durch ein auf das FPCB aufgebrachtes EMV-Muster realisiert sein. Diese Maßnahme verbessert die elektromagnetische Verträglichkeit (EMV) des Systems und schützt sowohl die eigenen Signale als auch benachbarte drahtlose Geräte vor Beeinträchtigungen.

In einem weiteren Aspekt wird vorgeschlagen, dass der Patch unmittelbar nach seiner Befestigung an der Armbanduhr eine automatische Kalibriersequenz der Vitalfunktionssensoren durchführt. Dabei werden initiale Basiswerte erfasst, die als Referenz für die nachfolgenden Messungen dienen. Diese automatische Kalibrierung erhöht die Genauigkeit und Verlässlichkeit der erfassten Daten und stellt eine konstante Messqualität bei wechselnden Tragesituationen sicher.

In einem weiteren Aspekt wird vorgeschlagen, dass der Patch in einem transparenten oder transluzenten Silikonmaterial vollständig oder teilweise eingebettet ist. Das Silikonmaterial ist vorzugsweise derart ausgewählt, dass es eine optische Schnittstelle zur drahtlosen Datenübertragung mittels Lichtsignalen, insbesondere im infraroten oder sichtbaren Spektralbereich, ermöglicht. Das transparente oder transluzente Silikon fungiert dabei vorzugsweise nicht nur als mechanischer und hautfreundlicher Schutzkörper, sondern auch als lichtleitendes Medium, das eine optische Kommunikation zwischen dem Patch und externen Geräten, wie beispielsweise tragbaren Diagnosesystemen oder Smartphone-Kameras, gestattet. Diese Kombination aus Materialeigenschaften erlaubt eine medienlose Signalübertragung, insbesondere auf Basis reflektiver oder direkter optischer Kopplung durch integrierte Photodioden, LEDs oder optische Sensoren im Inneren des Patches.

Die beschriebenen Ausgestaltungen und Weiterbildungen lassen sich beliebig miteinander kombinieren.

Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung.

### Kurze Beschreibung der Zeichnungen

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung.

Andere Ausführungsformen und viele der genannten Vorteile ergeben sich im Hinblick auf die Zeichnungen. Die dargestellten Elemente der Zeichnungen sind nicht notwendigerweise maßstabsgetreu zueinander gezeigt.
- Fig. 1: zeigt eine schematische Ansicht einer Armbanduhr mit einem Ausführungsbeispiel eines Patches.
- Fig. 2: zeigt eine schematische Ansicht einer Armbanduhr mit einem weiteren Ausführungsbeispiel eines Patches.
- Fig. 3: zeigt eine schematische Ansicht eines Ausführungsbeispiels eines Patches.
- Fig. 4: zeigt ein schematisches Flussdiagramm eines Ausführungsbeispiels eines vorliegenden Verfahrens.

### Detaillierte Beschreibung der Zeichnungen

In den Figuren der Zeichnungen bezeichnen gleiche Bezugszeichen gleiche oder funktionsgleiche Elemente, Bauteile oder Komponenten, soweit nichts Gegenteiliges angegeben ist.

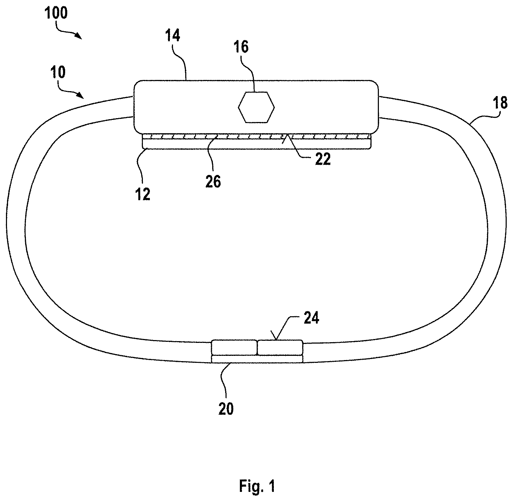

Fig. 1 zeigt ein Kit 100 mit einer Armbanduhr 10 und einem reversibel lösbar an der Armbanduhr 10 befestigten Patch 12. Das Kit 100 kann alternativ oder ergänzend zu der Armbanduhr 10 auch ein Armband aufweisen. Die Armbanduhr umfasst ein Uhrengehäuse 14, eine Lünette 16, ein Uhrenband 18 und eine an dem Uhrenband 18 angeordnete Schließe 20 zum Schließen des Uhrenbandes 18 um ein Handgelenk eines Benutzers. Das Uhrengehäuse 14 weist eine zum Handgelenk des Benutzers weisende Innenseite 22 auf. Die Schließe 20 weist eine zum Handgelenk des Benutzers weisende Innenseite 24 auf, die der Innenseite 22 gegenüberliegt. Die Armbanduhr 10 weist nur beispielhaft eine Lünette 16 auf. Die Armbanduhr 10 weist vorzugsweise ein mechanisches Uhrwerk auf (nicht gezeigt), so dass es sich bei der Armbanduhr 10 nicht um eine Smart-Watch, sondern um eine "klassische" Armbanduhr, beispielsweise eine Luxusuhr, handelt.

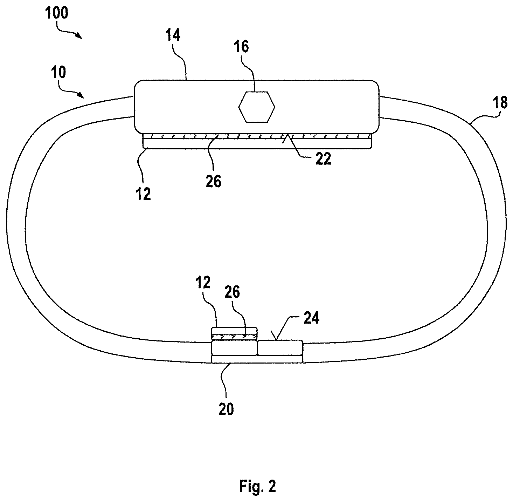

Der Patch 12 weist Mittel 26 zum reversibel lösbaren Befestigen des Patchs 12 an der Armbanduhr 10 auf. Vorliegend sind die Mittel zum Befestigen 26 als eine Klebeschicht bzw. -beschichtung oder als eine Haftschicht bzw. -beschichtung an dem Patch 10 vorgesehen. Gemäß Fig. 1 ist der Patch an der Innenseite 22 des Uhrenkörpers 14 reversibel lösbar befestigt. Gemä0 Fig. 2 weist das Kit 100 hingegen zwei Patches 12 auf. Einer der Patches 12 ist wie in Fig. 1 an der Innenseite 22 des Uhrenkörpers 14 reversibel lösbar befestigt. Der andere Patch 12 ist an einem Bereich der Innenseite 24 der Schließe 20 befestigt. Der Patch 12 kann in anderen Ausführungen auch nur an der Innenseite 24 der Schließe 20 befestigt sein. Der Patch 12 kann in anderen Ausführungen auch an anderen, vorzugsweise zum Handgelenk des Benutzers weisenden Bereichen der Armbanduhr 10 bzw. eines Armbandes befestigt sein. Der Patch 12 kann auch magnetisch und/oder mechanisch oder auf sonstige Weise reversibel lösbar befestigt sein. Die Innenseite 22 entspricht einer Handgelenkaußenseite. Die Innenseite 24 entspricht einer Handgelenkinnenseite.

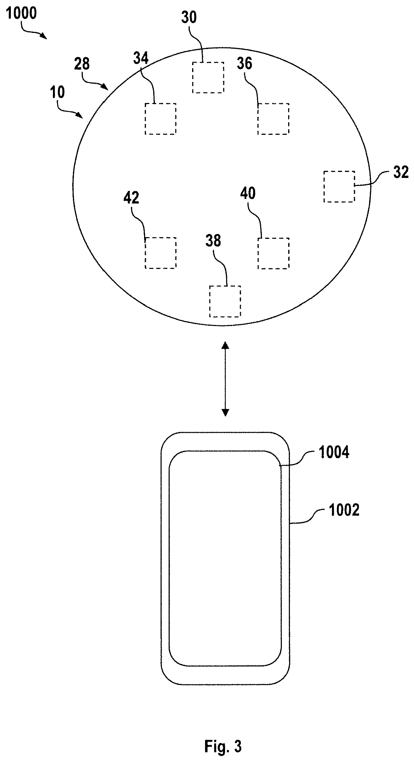

Ein Ausführungsbeispiel des Patches 12 ist in Fig. 3 näher gezeigt. Der Patch 12 ist dabei ein Teil eines Systems 1000, das ein externes Endgerät 1002 mit einer Benutzeroberfläche 1004 aufweist.

Der Patch 12 ist dabei als ein System on a Chip (SoC) 28. Eine derartige Ausbildung als SoC kann durch Fertigung des Patchs als ein PCB oder ein FPCB erfolgen. Der SoC kann ein- oder mehrschichtig sein.

Der Patch 12 umfasst mindestens einen Sensor 30 zur Erfassung mindestens einer Vitalfunktion des Benutzers. Der Patch 12 umfasst ferner mindestens einen weiteren Sensor 32 zur Erfassung einer geografischen Position und/oder mindestens einer Beschleunigung des Benutzers. Mindestens einer der Sensoren 30, 32 weist einen Herzfrequenzsensor und/oder einen Blutsauerstoffsättigungssensor und/oder einen Beschleunigungssensor und/oder mindestens eine Elektrode und einen Verstärker und/oder mindestens eine Photodiode und/oder einen Haut-/Gewebeimpedanzsensor auf. Alternativ oder ergänzend weist der Sensor 32 einen Standortsensor, insbesondere ein GPS-Modul, und/oder einen Beschleunigungssensor auf.

Der Patch 12 bzw. das SoC kann ferner eine Verarbeitungseinheit 34, eine Speichereinheit 36 und/oder eine Kommunikationseinheit 38 und/oder eine Energieversorgungseinheit 40 aufweisen. Der Patch 12 ist vorzugsweise autonom und/oder drahtlos energetisch versorgbar. Der Patch 12 kann alternativ oder ergänzend auch Mittel zum Aufladen 42 der internen Energieversorgungseinheit 40 aufweisen. Der Patch 12 ist dazu eingerichtet, über einen Energietransfer von der Armbanduhr 10 und/oder dem Armband betreibbar zu sein. Alternativ oder ergänzend ist der Patch 12 dazu eingerichtet, seine Energie durch Energyharvesting von der Armbanduhr 12 und/oder dem Armband und/oder aus einer Umgebung zu beziehen.

Der Patch 12 umfasst die insbesondere drahtlose Kommunikationseinheit 38, um Daten an das externe Gerät 1002 zu senden und/oder von dem externen Gerät 1002 zu empfangen, wobei das externe Gerät 1002 ein Mobiltelephon und/oder eine Cloud und/oder ein Server und/oder eine mobil Rechen- und/oder Steuereinrichtung umfassen kann.

In einer alternativen Ausführungsform kann der Patch 12 auch in dem Uhrenband 18 zumindest teilweise integriert sein. Ein derart ausgebildetes Uhrenband 18 kann dann an dem Uhrengehäuse 14 befestigt werden, um derart eine klassische Uhr bzw. Luxusuhr zumindest mit einer Vitalüberwachungsfunktion auszustatten, ohne dabei ein äußeres Erscheinungsbild der Uhr zu ändern. Selbiges gilt auch für ein Armband eines Schmuckarmbandes, bei dem ein Schmuckkörper, der beispielsweise Edelsteine oder ähnliches umfasst, mit einem daran anbringbaren Armband ausgestattet werden kann, in dem der vorliegend beschriebene Patch 12 zumindest teilweise integriert sein kann.

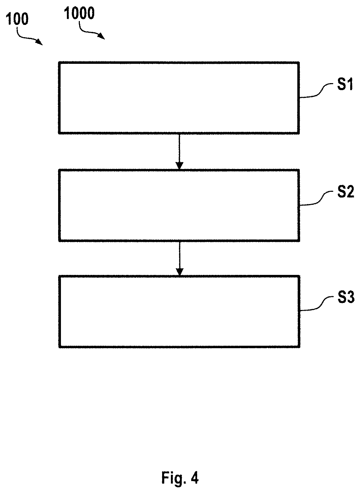

In Fig. 4 ist ein schematisches Flussdiagramm eines Verfahrens zur Überwachung von mindestens einer Vitalfunktion und/oder einem Standort eines Benutzers gezeigt.

In einem Schritt S1 erfolgt ein Bereitstellen mindestens eines Patches 12 an einer Armbanduhr 10 und/oder einem Armband des Benutzers.

In einem Schritt S2 erfolgt ein Erfassen der mindestens einen Vitalfunktion und/oder des Standortes des Benutzers durch den mindestens einen Patch 12.

In einem Schritt S3 erfolgt ein Senden der erfassten Daten an ein externes Gerät 1002.

### Bezugszeichenliste

- 10: Armbanduhr
- 12: Patch
- 14: Uhrengehäuse
- 16: Lünette
- 18: Uhrenband
- 20: Schließe
- 22: Innenseite
- 24: Innenseite
- 26: Mittel zur Befestigung
- 28: System on a Chip
- 30: Sensor
- 32: Sensor
- 34: Verarbeitungseinheit
- 36: Speichereinheit
- 38: Kommunikationseinheit
- 40: Energieversorgungseinheit
- 42: Mittel zum Aufladen
- 100: Kit
- 1000: System
- 1002: externes Endgerät
- 1004: Benutzeroberfläche

## Patentansprüche

1. Reversibel lösbar befestigbarer Patch (12) für eine Armbanduhr (10) und/oder ein Armband eines Benutzers, umfassend:
Mittel zur insbesondere reversiblen Befestigung (26) an der Armbanduhr (10) und/oder dem Armband;
mindestens einen Sensor (30) zur Erfassung mindestens einer Vitalfunktion des Benutzers; und vorzugsweise
mindestens einen weiteren Sensor (32) zur Erfassung einer geografischen Position und/oder mindestens einer Beschleunigung des Benutzers.

2. Patch nach Anspruch 1, wobei das Mittel zur Befestigung (26) durch einen Klettverschluss und/oder magnetisch und/oder mechanisch und/oder adhäsiv und/oder kohäsiv an der Armbanduhr (10) und/oder dem Armband befestigbar ist.

3. Patch nach Anspruch 1 oder 2, wobei der mindestens eine Sensor (30) in mindestens zwei elektrisch voneinander getrennten Sensoreinheiten segmentiert sind, die in unterschiedlichen Ebenen eines mehrschichtigen Flexible Printed Circuit Board (FPCB) angeordnet sind.

4. Patch nach Anspruch 3, wobei das Flexible Printed Circuit Board (FPCB) eine Biegeflexibilität von höchstens 0,5 N·mm und eine Rückstellkraft von mindestens 70 % innerhalb von fünf Sekunden nach Verformung aufweist.

5. Patch nach einem der vorhergehenden Ansprüche, wobei der Patch (12) autonom und/oder drahtlos energetisch versorgbar ist, und/oder wobei der Patch (12) Mittel zum Aufladen (42) einer internen Energieversorgungseinheit aufweist.

6. Patch nach einem der vorhergehenden Ansprüche, wobei der Patch (12) ein thermisch reaktives Material aufweist, das in Abhängigkeit von der Hauttemperatur des Benutzers seine Form oder Elastizität verändert,

7. Patch nach einem der vorhergehenden Ansprüche, wobei der Patch (12) ein kapazitives oder optisches System zur automatischen Erkennung des Tragens auf der Haut aufweist und in einem inaktiven Energiesparmodus verbleibt, solange keine Hautdetektion erfolgt.

8. Patch nach einem der vorhergehenden Ansprüche, wobei mindestens einer der Sensoren (30, 32) einen Herzfrequenzsensor und/oder einen Blutsauerstoffsättigungssensor und/oder einen Beschleunigungssensor und/oder mindestens eine Elektrode und einen Verstärker und/oder mindestens eine Photodiode und/oder einen Haut-/Gewebeimpedanzsensor aufweist, und/oder wobei der mindestens eine weitere Sensor (32) einen Standortsensor, insbesondere ein GPS-Modul, und/oder einen Beschleunigungssensor aufweist.

9. Patch nach einem der vorhergehenden Ansprüche, wobei der Patch (12) innenseitig an der Armbanduhr (12) und/oder dem Armband, insbesondere in Richtung einer Handgelenkinnenseite (24) und/oder einer Handgelenkaußenseite (24) befestigbar ist.

10. Patch nach einem der vorhergehenden Ansprüche, wobei der Patch (12) dazu eingerichtet ist, über einen Energietransfer von der Armbanduhr (10) und/oder dem Armband betreibbar zu sein, oder wobei der Patch (12) dazu eingerichtet ist, seine Energie durch Energy-Harvesting von der Armbanduhr (10) und/oder dem Armband zu beziehen.

11. Patch nach einem der vorhergehenden Ansprüche, wobei der Patch (12) ferner eine drahtlose Kommunikationseinheit (38) umfasst, um Daten an ein externes Gerät (1002) zu senden und/oder von dem externen Gerät (1002) zu empfangen, wobei das externe Gerät (1002) ein Mobiltelephon und/oder eine Cloud und/oder ein Server und/oder eine mobil Rechen- und/oder Steuereinrichtung umfassen kann.

12. Patch (12) für eine Armbanduhr (10) oder ein Schmuckarmband, wobei der Patch (12) zumindest teilweise in einem reversibel lösbar befestigbaren Uhrenband (18) der Armbanduhr (10) oder einem Armband eines Schmuckarmbandes integriert ist, der Patch (12) umfassend:
Mittel zur insbesondere reversiblen Befestigung (26) an der Armbanduhr (10);
mindestens einen Sensor (30) zur Erfassung mindestens einer Vitalfunktion des Benutzers; und vorzugsweise
mindestens einen weiteren Sensor (32) zur Erfassung einer geografischen Position und/oder mindestens einer Beschleunigung des Benutzers.

13. Kit (100), umfassend den Patch (12) nach einem der Ansprüche 1 bis 12 und eine Armbanduhr (10) und/oder ein Armband, an der/dem der Patch (12) reversibel befestigbar ist.

14. System (1000), umfassend:
a) den Patch (12) nach einem der Ansprüche 1 bis 11; und
b) ein externes Gerät (1002), das dazu ausgebildet ist, die von dem Patch (12) gesendeten Daten zu empfangen und/oder zu verarbeiten.

15. System (1000) nach Anspruch 14, wobei das externe Gerät (1002) eine Benutzeroberfläche (1004) umfasst, die dem Benutzer ermöglicht, die Funktionalität des Patches (12) zu steuern oder die erfassten Daten anzuzeigen.
